# EUROPEAN PATENT APPLICATION

(11) **EP 2 615 182 A1**
(43) Date of publication of application: **17.07.2013**
(21) Application number: 13150799.8
(22) Date of filing: 10.01.2013
(51) Int. Cl.: C12Q 1/68

(54) **Method for detecting PON1 gene polymorphism (Q192R)**

(30) Priority: 10.01.2012 JP 2012002597
(71) Applicant: ARKRAY, Inc., Kyoto-shi, Kyoto 601-8045 (JP)
(72) Inventor: Kurose, Kaoru, Kyoto-shi, Kyoto 602-0008 (JP); Komori, Mariko, Kyoto-shi, Kyoto 602-0008 (JP)
(74) Representative: Jones, Elizabeth Louise

(57) **Abstract**

A probe for detecting a polymorphism in the PON1 gene, comprising at least one fluorescently labeled oligonucleotide selected from P1, P1', P2 and P2' below: (P1) an oligonucleotide comprising a nucleotide sequence of 6 to 53 consecutive nucleotides containing nucleotides 299 to 304 in SEQ ID NO: 1 or a homologous sequence thereof, wherein the nucleotide corresponding to the nucleotide at position 304 is a cytosine and is labeled with a fluorescent dye; (P1') an oligonucleotide comprising a nucleotide sequence of 6 to 53 consecutive nucleotides containing nucleotides 299 to 304 in SEQ ID NO: 1 or a nucleotide sequence which hybridizes with the complementary strand of the nucleotides 299 to 304 in SEQ ID NO: 1 under stringent conditions, wherein the nucleotide corresponding to the nucleotide at position 304 is a cytosine and is labeled with a fluorescent dye; (P2) an oligonucleotide comprising a nucleotide sequence of 12 to 54 consecutive nucleotides containing nucleotides 290 to 301 in SEQ ID NO: 1 or a homologous sequence thereof, wherein the nucleotide corresponding to the nucleotide at position 290 is a cytosine and is labeled with a fluorescent dye; and (P2') an oligonucleotide comprising a nucleotide sequence of 12 to 54 consecutive nucleotides containing nucleotides 290 to 301 in SEQ ID NO:1 or a nucleotide sequence which hybridizes with the complementary strand of the nucleotides 290 to 301 in SEQ ID NO: 1 under stringent conditions, wherein the nucleotide corresponding to the nucleotide at position 290 is a cytosine and is labeled with a fluorescent dye.

SEQUENCE LISTING

<110>  Arkray, Inc.

<120>  METHOD FOR DETECTING PON1 GENE POLYMORPHISM (Q192R)

<130>  42.13.115166

<150>  JP2012-002597
<151>  2012-01-10

<160>  28

<170>  PatentIn version 3.5

<210>  1
<211>  601
<212>  DNA
<213>  Homo sapiens

<400>  1
tacagtgcta taatcacctc ctcagatttc ttagaccaat gattgtcttt aaggattgta      60

tcggcaggac taatttcata ttaattgtgt tccattatag ctagcacgaa ggctccatcc     120

catatcttga ttttaggaat agacagtgag gaatgccagt taataatcct gtaatgttca     180

ataccttcac cttatatatt atgtgtgtat gttttaattg cagtttgaat gatattgttg     240

ctgtgggacc tgagcacttt tatggcacaa atgatcacta ttttcttgac ccctacttac     300

ratcctggga gatgtatttg ggtttagcgt ggtcgtatgt tgtctactat agtccaagtg     360

aagttcgagt ggtggcagaa ggatttgatt ttgctaatgg aatcaacatt tcacccgatg     420

gcaagtatgt gaactctctg aaatgtagtg gatttactca gattctcagg agatatcttg     480

gaatatattc tttttttaag taatatgata cattttaaca tgttacccta gtgagataaa     540

attaggaaaa atgtaaaatg tcttaatttt tcagggggtg aaatttatca aatcaaaaat     600

t                                                                     601


<210>  2
<211>  18
<212>  DNA
<213>  Artificial Sequence

<220>
<223>  probe

<400>  2
tgacccctac ttacgatc                                                    18


<210>  3
<211>  28
<212>  DNA
<213>  Artificial Sequence

<220>
<223>  probe

<400>  3
   actattttct tgacccctac ttacgatc                                    28


<210>  4
<211>  38
<212>  DNA
<213>  Artificial Sequence

<220>
<223>  probe

<400>  4
acaaatgatc actattttct tgacccctac ttacgatc                         38


<210>  5
<211>  48
<212>  DNA
<213>  Artificial Sequence

<220>
<223>  probe

<400>  5
cttttatggc acaaatgatc actattttct tgacccctac ttacgatc              48


<210>  6
<211>  53
<212>  DNA
<213>  Artificial Sequence

<220>
<223>  probe

<400>  6
gagcactttt atggcacaaa tgatcactat tttcttgacc cctacttacg atc        53


<210>  7
<211>  54
<212>  DNA
<213>  Artificial Sequence

<220>
<223>  probe

<400>  7
tgagcacttt tatggcacaa atgatcacta ttttcttgac ccctacttac gatc        54


<210>  8
<211>  10
<212>  DNA
<213>  Artificial Sequence

<220>
<223>  probe

<400>  8
acttacgatc                                                          10


<210>  9
<211>  12

<212>  DNA
<213>  Artificial Sequence

<220>
<223>  probe

<400>  9
cccctactta ca                                                          12


<210>  10
<211>  18
<212>  DNA
<213>  Artificial Sequence

<220>
<223>  probe

<400>  10
cccctactta caatcctg                                                    18


<210>  11
<211>  28
<212>  DNA
<213>  Artificial Sequence

<220>
<223>  probe

<400>  11
ccctacttac aatcctggga gatgtatt                                         28


<210>  12
<211>  38
<212>  DNA
<213>  Artificial Sequence

<220>
<223>  probe

<400>  12
ccctacttac aatcctggga gatgtatttg ggtttagc                              38


<210>  13
<211>  48
<212>  DNA
<213>  Artificial Sequence

<220>
<223>  probe

<400>  13
ccctacttac aatcctggga gatgtatttg ggtttagcgt ggtcgtat                   48


<210>  14
<211>  54
<212>  DNA
<213>  Artificial Sequence

<220>
<223>  probe

<400>  14
ccctacttac aatcctggga gatgtatttg ggtttagcgt ggtcgtatgt tgtc          54


<210>  15
<211>  55
<212>  DNA
<213>  Artificial Sequence

<220>
<223>  probe

<400>  15
ccctacttac aatcctggga gatgtatttg ggtttagcgt ggtcgtatgt tgtct         55


<210>  16
<211>  21
<212>  DNA
<213>  Artificial Sequence

<220>
<223>  primer

<400>  16
gttgctgtgg gacctgagca c                                              21


<210>  17
<211>  25
<212>  DNA
<213>  Artificial Sequence

<220>
<223>  primer

<400>  17
tctgccacca ctcgaacttc acttg                                          25


<210>  18
<211>  25
<212>  DNA
<213>  Artificial Sequence

<220>
<223>  oligonucleotide

<400>  18
cccaggattg taagtagggg tcaag                                          25


<210>  19
<211>  25
<212>  DNA
<213>  Artificial Sequence

<220>
<223>  oligonucleotide

<400>  19
cccaggatcg taagtagggg tcaag                                          25

<210>  20
<211>  18
<212>  DNA
<213>  Artificial Sequence

<220>
<223>  probe

<400>  20
cttgacccct acttacga                                                    18


<210>  21
<211>  399
<212>  DNA
<213>  Homo sapiens

<400>  21
taatcagaga atactacaca tgtacaataa aaatttcccc atcaagatat acaatatatt      60

ttatttatat ttatagtttt aaattacaac cagagcttgg catattgtat ctataccttt     120

attaaatgct tttaatttaa taaattattg ttttctctta gatatgcaat aattttccca     180

ctatcattga ttatttcccr ggaacccata acaaattact taaaaacctt gcttttatgg     240

aaagtgatat tttggagaaa gtaaaagaca ccaagaatcg atggacatca acaaccctcg     300

ggactttatt gattgcttcc tgatcaaaat ggagaaggta aaatgttaac aaaagcttag     360

ttatgtgact gcttgcgtat ttgtgattca ttgactagt                            399


<210>  22
<211>  40
<212>  DNA
<213>  Artificial Sequence

<220>
<223>  primer

<400>  22
taaattattg ttttctctta gatatgcaat aattttccca                            40


<210>  23
<211>  23
<212>  DNA
<213>  Artificial Sequence

<220>
<223>  primer

<400>  23
cccgagggtt gttgatgtcc atc                                              23


<210>  24
<211>  17
<212>  DNA
<213>  Artificial Sequence

<220>
<223>  probe

<400>  24
tcccaggaac ccataac                                                    17


<210>  25
<211>  600
<212>  DNA
<213>  Homo sapiens

<400>  25
atgcatgcca aactcttttt tgcttttaag ggaattcata ggtaagatat tacttaaaat      60

ttctaaacta ttattatctg ttaacaaata tgaagtgttt tatatctaat gtttactcat     120

attttaaaat tgtttccaat catttagctt caccctgtga tcccactttc atcctgggct     180

gtgctccctg caatgtgatc tgctccatta ttttccagaa acgtttcgat tataaagatc     240

agcaatttct taacttgatg gaaaaattga atgaaaacat caggattgta agcaccccct     300

gratccaggt aaggccaagt tttttgcttc ctgagaaacc acttacagtc tttttttctg     360

ggaaatccaa aattctatat tgaccaagcc ctgaagtaca tttttgaata ctacagtctt     420

gcctagacag ccatggggtg aatatctgga aaagatggca aagttcttta ttttatgcac     480

aggaaatgaa tatcccaata tagatcaggc ttctaagccc attagctccc tgatcagtgt     540

tttttccact aaactccaaa gccctgtttc tataaagtac tttggtgaca gccccaaagc     600


<210>  26
<211>  35
<212>  DNA
<213>  Artificial Sequence

<220>
<223>  primer

<400>  26
tgatggaaaa attgaatgaa aacatcagga ttgta                                35


<210>  27
<211>  30
<212>  DNA
<213>  Artificial Sequence

<220>
<223>  primer

<400>  27
tcaaaaatgt acttcagggc ttggtcaata                                      30


<210>  28
<211>  17
<212>  DNA
<213>  Artificial Sequence

<220>
<223>  probe

<400>  28
ccctgaatcc aggtaag                                                    17

## Description

### TECHNICAL FIELD

The present invention relates to a method for detecting a PON1 gene polymorphism (Q192R), a nucleic acid probe and a kit therefor.

### BACKGROUND ART

The PON (paraoxanase) gene cluster is present in the q21.3-q22.1 region on chromosome 7 and encodes three paraoxanase isoforms PON1, PON2 and PON3 having similar structures (The 2 1 st International Symposium on ALS/MND, "Paraoxonase Gene Mutations in Amyotrophic Lateral Sclerosis", 2010, Enlightenment Committee on ALS Disease, internet <URL:
http://www.als.gr.jp/staff/sympo/21st_sympo/sympo21_11.html>). Although the three enzymes share common functions to protect against hyperoxidation of membrane lipids, each of them also has a specific function. PON1 detoxifies organophosphates and PON2 protects cells against endoplasmic reticulum stress. However, the physiological function of PON3 has not yet been sufficiently elucidated (The 21st International Symposium on ALS/MND, "Paraoxonase Gene Mutations in Amyotrophic Lateral Sclerosis", 2010, Enlightenment Committee on ALS Disease, internet <URL: http://www.als.gr.jp/staff/sympo/21st_sympo/sympo21_11.html>).

The PON1 protein in blood is bound to apoA-1 ofHDL (high density lipoprotein), and the protein is considered to inhibit the oxidation of LDL (low density lipoprotein) and suppress the progress of arteriosclerosis (J Clin Invest 96(6): 3005-8, 1995). Further, involvement of PON1 (Q192R) in myocardial infarction and coronary spasm has been suggested (J Clin Invest 96(6): 3005-8, 1995).
Further, PON1 was identified as the most important enzyme in the activation of the antiplatelet drug Plavix (generic name: Clopidogrel), and its gene polymorphism Q192R has been revealed to be a determinant of conversion efficiency into the active form (Nature Medicine 17, 110-116 (2011)). Since it is an antithrombotic drug used for stent therapy, Clopidogrel is commonly used. In Nature Medicine 17, 110-116 (2011), the clinical significance of the PON1 Q192R gene polymorphism was investigated in patients with coronary artery disease undergoing stent placement and receiving administration of Clopidogrel. The results suggested that PON1 QQ192 homozygous patients show, when compared to RR192 homozygous patients, higher risk of occurrence of stent thrombosis, lower PON1 activity and Clopidogrel active metabolite in serum, and insufficient inhibition of platelet activity. Thus, it has been reported that the pharmacological effect of Clopidogrel can be hardly obtained in PON1 QQ192 homozygous patients.

In Nature Medicine 17, 110-116 (2011), the presence/absence of the PON1 (Q192R) mutation was detected using sequence analysis. Extraction of genomic DNA from whole blood for investigation of the pharmacological effect of Clopidogrel is very labourious and costly at actual clinical sites.

JP 2002-119291 A describes a method wherein a nucleic acid probe labeled with a fluorescent dye is allowed to hybridize with a target nucleic acid and the amount of decrease in the luminescence from the fluorescent dye is measured. However, in cases where a nucleic acid probe labeled with a fluorescent dye is allowed to hybridize with a target nucleic acid and the amount of decrease in the luminescence from the fluorescent dye is measured, the sequence of the probe cannot be arbitrary and an appropriate sequence must be found for each mutation.

In Pharmacogenetics 4, 1994, pp. 285-299, it is reported that the presence of the homozygous CYP2C19*2 mutation causes splicing abnormality, and this leads to an abnormal protein structure, resulting in decreased metabolic efficiencies of various drugs and increased risks of side effects and the like. In Molecular Pharmacology 46, 1994, pp. 594-598, it is reported that the presence of the homozygous CYP2C19*3 mutation results in mutation to a stop codon and hence in production of a protein having an abnormal structure by translation, which then leads to decreased metabolic efficiencies of various drugs and increased risks of side effects and the like. Thus, since not only PON1 (Q192R) but also CYP2C19*2 and CYP2C19*3 are involved in the pharmacological effect of Clopidogrel, it is likely that a technique that enables the simultaneous measurement of the presence/absence of PON1 (Q192R) as well as CYP2C19*2 and CYP2C19*3 will be in greater demand in clinical settings.

In Pharmacogenetics 4, 1994, pp. 285-299, the presence/absence of the CYP2C19*2 mutation was detected using PCR-RFLP. In Molecular Pharmacology 46, 1994, pp. 594-598, the presence/absence of the CYP2C19*3 mutation was detected using PCR-RFLP. In JP 2005-58114 A and WO2008/066162, methods for detecting the CYP2C19*2 mutation and nucleic acid probes therefor are described. In JP 2005-110502 A, a method for detecting the CYP2C19*3 mutation and nucleic acid probes therefor are described.

However, none of the above-described prior art documents discloses a simple method for measuring the presence/absence of the PON1 (Q192R) mutation involved in the pharmacological effect of Clopidogrel and a probe to be used therefor, or a method for simultaneously measuring the presence/absence of the PON1 (Q192R) as well as CYP2C19*2 and CYP2C19*3 mutations involved in the pharmacological effect of Clopidogrel.

### SUMMARY OF THE INVENTION

The present invention aims to provide probes effective for detection of a PON1 gene polymorphism (Q192R), a method for detecting the PON1 gene polymorphism (Q192R), and a method for simultaneously detecting the three mutations PON1 (Q192R), CYP2C19*2 and CYP2C19*3, and a kit therefor.

The present inventors discovered that, by designing a probe based on a specific region containing the PON1 gene polymorphism (Q192R) and using the probe for detecting a signal due to formation of a hybrid of the probes with target nucleic acids or due to dissociation of the probes from target nucleic acids, the mutation can be detected, thereby completing the present invention.

That is, the present invention is as follows:
(1) A probe for detecting a polymorphism in the PON1 gene, comprising at least one fluorescently labeled oligonucleotide selected from P1, P1', P2 and P2' below:
   (P1) an oligonucleotide comprising a nucleotide sequence of 6 to 53 consecutive nucleotides containing nucleotides 299 to 304 in SEQ ID NO: 1 or a homologous sequence thereof, wherein the nucleotide corresponding to the nucleotide at position 304 is a cytosine and is labeled with a fluorescent dye;
   (P1') an oligonucleotide comprising a nucleotide sequence of 6 to 53 consecutive nucleotides containing nucleotides 299 to 304 in SEQ ID NO: 1 or a nucleotide sequence which hybridizes with the complementary strand of the nucleotides 299 to 304 in SEQ ID NO: 1 under stringent conditions, wherein the nucleotide corresponding to the nucleotide at position 304 is a cytosine and is labeled with a fluorescent dye;
   (P2) an oligonucleotide comprising a nucleotide sequence of 12 to 54 consecutive nucleotides containing nucleotides 290 to 301 in SEQ ID NO: 1 or a homologous sequence thereof, wherein the nucleotide corresponding to the nucleotide at position 290 is a cytosine and is labeled with a fluorescent dye; and
   (P2') an oligonucleotide comprising a nucleotide sequence of 12 to 54 consecutive nucleotides containing nucleotides 290 to 301 in SEQ ID NO:1 or a nucleotide sequence which hybridizes with the complementary strand of the nucleotides 290 to 301 in SEQ ID NO: 1 under stringent conditions, wherein the nucleotide corresponding to the nucleotide at position 290 is a cytosine and is labeled with a fluorescent dye.
(2) The probe according to (1), wherein
   said oligonucleotides P1 and P1' have the nucleotide corresponding to the nucleotide at position 304 labeled with a fluorescent dye at the first, second or third position counted from the 3'-end; and
   said oligonucleotides P2 and P2' have the nucleotide corresponding to the nucleotide at position 290 labeled with a fluorescent dye at the first, second or third position counted from the 5'-end.
(3) The probe according to (1) or (2), wherein
   said oligonucleotides P1 and P1' have the nucleotide corresponding to the nucleotide at position 304 labeled with a fluorescent dye at the 3'-end; and
   said oligonucleotides P2 and P2' have the nucleotide corresponding to the nucleotide at position 290 labeled with a fluorescent dye at the 5'-end.
(4) The probe according to any one of (1) to (3), wherein said fluorescently labeled oligonucleotide emits fluorescence when the fluorescently labeled oligonucleotide is not hybridized with a target sequence, and the fluorescence intensity decreases or increases when the fluorescently labeled oligonucleotide is hybridized with a target sequence.
(5) The probe according to (4), wherein said fluorescently labeled oligonucleotide emits fluorescence when the fluorescently labeled oligonucleotide is not hybridized with a target sequence, and the fluorescence intensity decreases when the fluorescently labeled oligonucleotide is hybridized with a target sequence.
(6) The probe according to any one of (1) to (5), wherein
   said oligonucleotides P1 and P1' have 6 to 28 consecutive nucleotides; and
   said oligonucleotides P2 and P2' have 12 to 28 consecutive nucleotides.
(7) The probe according to any one of (1) to (5), wherein
   said oligonucleotides P1 and P1' have 6 to 18 consecutive nucleotides; and
   said oligonucleotides P2 and P2' have 12 to 18 consecutive nucleotides.
(8) The probe according to any one of (1) to (7), wherein said probe is a probe for melting curve analysis.
(9) A method for detecting a polymorphism in the PON1 gene using the probe according to any one of (1) to (8). The method may be conducted on a sample comprising nucleic acid which may contain said polymorphism and comprises contacting the probe with said sample and detecting the presence or absence of the polymorphism. The invention also extends to use of said probe for detecting said polymorphism.
(10) A method for detecting a polymorphism in the PON1 gene, comprising Steps (I) to (IV) below:
   (I) adding said probe according to any one of (1) to (8) to a sample containing DNA and allowing said probe to hybridize with said DNA;
   (II) changing the temperature to dissociate the hybridization complex between said DNA and said probe and measuring the fluctuation of the signal due to said dissociation of said hybridization complex;
   (III) analyzing said fluctuation of the signal to determine the Tm value; and
   (IV) determining based on said Tm value the presence of the polymorphism of interest.
(11) The method according to (10), further comprising amplifying nucleic acid before said Step (I) or at the same time as said Step (I).
(12) The method according to any one of (9) to (11), further comprising detecting in the same system (i.e. in the same reaction or same reaction tube) at least one polymorphism selected from a polymorphism of the nucleotide corresponding to the nucleotide at position 200 in SEQ ID NO:21 and a polymorphism of the nucleotide corresponding to the nucleotide at position 302 in SEQ ID NO:25.
(13) The method according to any one of (9) to (12), further comprising detecting in the same system a polymorphism of the nucleotide corresponding to the nucleotide at position 301 in SEQ ID NO:1, a polymorphism of the nucleotide corresponding to the nucleotide at position 200 in SEQ ID NO:21 and a polymorphism of the nucleotide corresponding to the nucleotide at position 302 in SEQ ID NO:25.
(14) A method for judging (or predicting) the pharmacological effect of a drug, comprising detecting a polymorphism in the PON1 gene by the method for detecting a polymorphism according to any one of (9) to (13) and judging (or predicting) resistance to said drug and/or the pharmacological effect of said drug based on the presence or absence of said polymorphism. Said method may be performed in a sample from a subject (e.g. a human) in relation to whom the judgement/prediction is to be made. A sample (as used herein) comprises nucleic acid (e.g. DNA) which may contain said polymorphism.
(15) A reagent kit for detecting a polymorphism in the PON1 gene, comprising the probe according to any one of (1) to (8).
(16) The kit according to (15), further comprising a primer for amplifying a nucleotide sequence comprising the region with which said probe hybridizes.
(17) The kit according to (15) or (16), further comprising:
   a polymorphism detection probe for detecting a polymorphism of the nucleotide corresponding to the nucleotide at position 200 in SEQ ID NO:21; and
   a polymorphism detection probe for detecting a polymorphism of the nucleotide corresponding to the nucleotide at position 302 in SEQ ID NO:25. The invention also extends to the use of said kit for detecting said polymorphism(s).

By including the probe of the present invention in a gene amplification system such as PCR and only carrying out Tm analysis after the gene amplification reaction, typing of the PON1 gene polymorphism (Q192R) can be carried out. Since the probe of the present invention has high specificity, it can be used also in cases where whole blood, an oral mucosa suspension or the like is directly tested. Accordingly, the labour and the costs can be reduced in clinical tests such as tests before dosing (i.e. before administering a drug).
By using the method of the present invention, even in cases where PCR is carried out, the necessity of removal of the amplification product can be eliminated. Therefore, there is hardly any risk of contamination. Further, since the method of the present invention can be carried out by a simple procedure, it can be easily automated.
By using the method of the present invention, it is also possible to simultaneously detect the three mutations PON1 (Q192R), CYP2C19*2 and CYP2C19*3. In cases where Clopidogrel is used in stent therapy, it is clinically important to be able to simultaneously detect the three mutations.

### BRIEF DESCRIPTION OF THE DRAWINGS

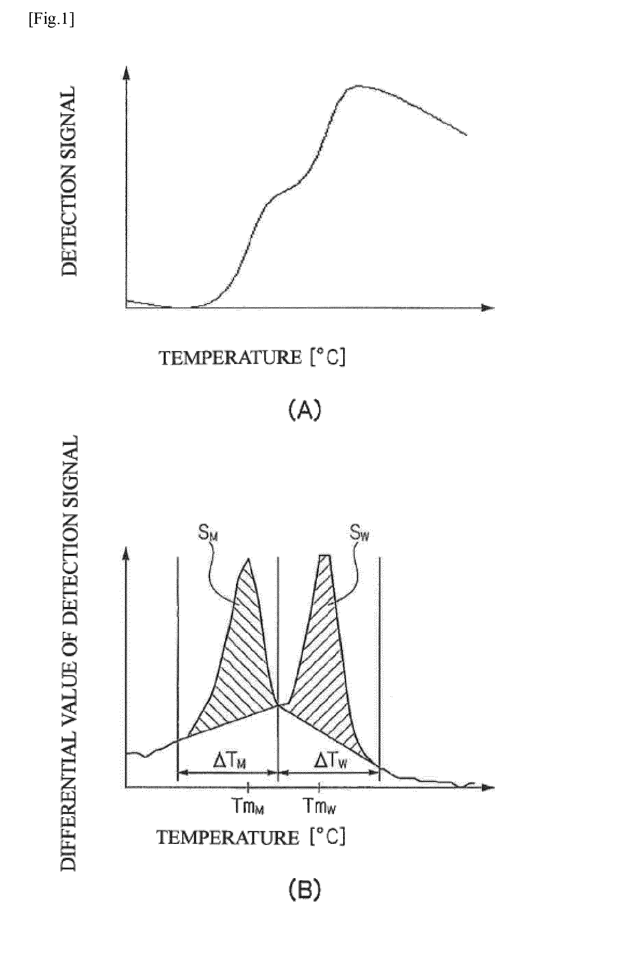

Fig. 1 shows diagrams showing examples of (A) a melting curve of a nucleic acid mixture; and (B) a differential melting curve.
Fig. 2 shows melting curves obtained using the probe of Example 1 of the present invention for detecting a polymorphism. The amount of change in fluorescence intensity per unit time is plotted along the ordinate (d the amount of increase in fluorescence intensity/t) and the temperature (°C) is plotted along the abscissa. Squares represent the wild-type, diamonds represent the mutant type, and triangles represent the heterozygous type (this also applies to the figures below).
Fig. 3 shows melting curves obtained using the probe of Example 2 of the present invention for detecting a polymorphism.
Fig. 4 shows melting curves obtained using the probe of Comparative Example 1 for detecting a polymorphism.
Fig. 5 shows melting curves obtained from purified human genomic DNA using the probes of Example 3 of the present invention for detecting polymorphisms. The left panel shows a melting curve for detecting the PON1 gene polymorphism (Q192R); the middle panel shows a melting curve for detecting the CYP2C19*2 gene polymorphism; and the right panel shows a melting curve for detecting the CYP2C19*3 gene polymorphism (this also applies to the figure below).
Fig. 6 shows melting curves obtained from whole blood using the probes of Example 3 of the present invention.

### MODE FOR CARRYING OUT THE INVENTION

### <1> Probes of the Present Invention and Detection Method of the Present Invention

The (P1) probe of the present invention is a probe for detecting the PON1 gene polymorphism (Q192R) (that is, rs662), and is composed of an oligonucleotide comprising a nucleotide sequence of 6 to 53 consecutive nucleotides containing nucleotides 299 to 304 in SEQ ID NO:1 or a homologous sequence thereof, wherein the nucleotide corresponding to the nucleotide at position 304 is a cytosine and is labeled with a fluorescent dye. As referred to in this context, the homologous sequence is homologous to SEQ ID NO:1 or the recited portion of SEQ ID NO:1. Thus, said oligonucleotide has 6 to 53 nucleotides derived from said homologous sequence, including nucleotides corresponding to nucleotides 299 to 304 in SEQ ID NO:1. The 6 to 53 nucleotides are all contained, consecutively in SEQ ID NO:1 or its homologous sequence.
The (P1') probe of the present invention is a probe for detecting the PON1 gene polymorphism (Q192R) (that is, rs662), and is composed of an oligonucleotide comprising a nucleotide sequence of 6 to 53 consecutive nucleotides containing nucleotides 299 to 304 in SEQ ID NO:1 or a nucleotide sequence which hybridizes with the complementary strand of the nucleotides 299 to 304 in SEQ ID NO: 1 under stringent conditions, wherein the nucleotide corresponding to the nucleotide at position 304 is a cytosine and is labeled with a fluorescent dye. As referred to in this context, the hybridizing sequence hybridizes with the complementary strand of SEQ ID NO:1 or the recited portion of SEQ ID NO:1. Thus, said oligonucleotide has 6 to 53 nucleotides derived from said hybridizing sequence, including nucleotides corresponding to nucleotides 299 to 304 in SEQ ID NO:1. The 6 to 53 nucleotides are all contained, consecutively in SEQ ID NO:1 or the hybridizing sequence.
The (P1) and (P1') probes of the present invention may also be peptide nucleic acid (PNA) having amide-linked N-(2-aminoethyl)glycine as the backbone instead of the deoxyribose backbone of an oligonucleotide, or may be nucleic acid having two cyclic structures wherein the oxygen atom at the 2'-position and the carbon atom at the 4'-position in the sugar of nucleic acid are cross-linked (LNA; Locked Nucleic Acid).
The PON1 gene polymorphism rs662 described herein is located at nucleotide position 301 in SEQ ID NO:1. The rs number represents a registration number for the dbSNP database curated by the National Center for Biotechnology Information (//www.ncbi.nlm.nih.gov/projects/SNP/). The nucleotide at position 301 in SEQ ID NO:1 is represented as r, which corresponds to the nucleotide A in the wild-type and the nucleotide G in the mutant type. For example, in the (P1) and (P1') probe of the present invention, the nucleotide at position 301 in SEQ ID NO:1 is G, which is the mutant-type nucleotide.
The (P1) and (P1') probes of the present invention can be prepared in the same manner as the probe described in JP 2002-119291 A except that the (P1) and (P1') probes of the present invention have the above-described specified sequence in the nucleotide sequence shown in SEQ ID NO:1. As shown in Table 1, the length of the (P1) and (P1') probe is, for example, 6 to 53 consecutive nucleotides, 10 to 30 consecutive nucleotides or 13 to 25 consecutive nucleotides. In the present invention, for example, the mutation of the nucleotide at position 301 in SEQ ID NO:1 can be more sensitively detected in cases where the difference between the Tm value observed when the (P1) oligonucleotide is hybridized with DNA having the complementary nucleotide sequence and the Tm value observed when the (P1) oligonucleotide is hybridized with DNA having the complementary nucleotide sequence except that only one nucleotide at position 301 in SEQ ID NO:1 is not complementary is not less than 2.0°C.
Further, as shown in Table 2, in cases where an artificial nucleic acid such as LNA is used, a probe having not less than 6 consecutive nucleotide is enough to obtain Tm values with which mutation of the nucleotide at position 301 in SEQ ID NO:1 can be detected.

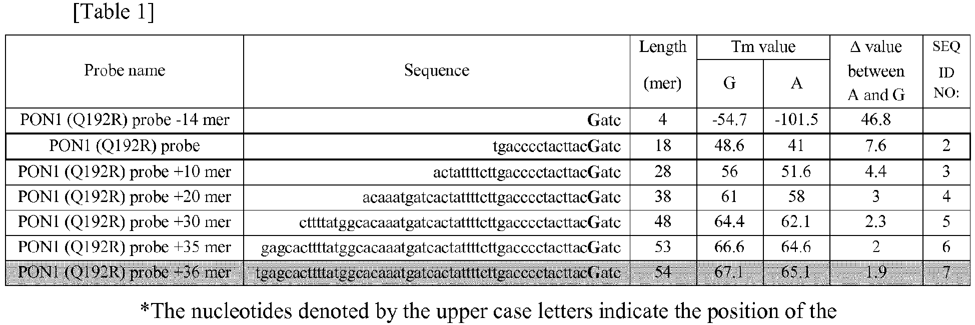

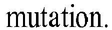

The Tm value was calculated with Meltcalc 99 free (http://www.meltcalc.com/) under the conditions of: Oligoconc [µM] 0.2, Na eq. [mM] 50.

**[Table 2]**

| | Sequence | Length (mer) | Tm value | SEQ ID NO: |
|---|---|---|---|---|
| | | | G | |
| PON1 (Q192R) probe using LNA | **G**atc | 4 | ? | |
| | ac**G**atc | 6 | 38 | |
| | ttac**G**atc | 8 | 57 | |
| | acttac**G**atc | 10 | 70 | 8 |

| | | | | |
|---|---|---|---|---|
| *The nucleotides denoted by the upper case letters indicate the position of the mutation. | | | | |

Table 2 describes the Tm values observed when LNA was used. By using LNA, the Tm value can be calculated even in cases of 6 consecutive nucleotides. The Tm value was calculated using LNA™ Oligo Tools and Design Guidelines (http://www.exiqon.com/oligo-tools).
Examples of the nucleotide sequence of the (P1) probe used in the present invention include 5'-tgacccctacttacGatc-3' (SEQ ID NO:2), in which the upper-case nucleotide G corresponds to the nucleotide at position 301.

The (P2) probe of the present invention is a probe for detecting the PON1 gene polymorphism (Q192R) (that is, rs662), and is composed of an oligonucleotide comprising a nucleotide sequence of 12 to 54 consecutive nucleotides containing nucleotides 290 to 301 in SEQ ID NO:1 or a homologous sequence thereof, wherein the nucleotide corresponding to the nucleotide at position 290 is a cytosine and is labeled with a fluorescent dye. As referred to in this context, the homologous sequence is homologous to SEQ ID NO:1 or the recited portion of SEQ ID NO:1. Thus, said oligonucleotide has 12 to 54 nucleotides derived from said homologous sequence, including nucleotides corresponding to nucleotides 290 to 301 in SEQ ID NO:1. The 12 to 54 nucleotides are all contained, consecutively in SEQ ID NO:1 or its homologous sequence.
The (P2') probe of the present invention is a probe for detecting the PON1 gene polymorphism (Q192R) (that is, rs662), and is composed of an oligonucleotide comprising a nucleotide sequence of 12 to 54 consecutive nucleotides containing nucleotides 290 to 301 in SEQ ID NO:1 or a nucleotide sequence which hybridizes with the complementary strand of the nucleotides 290 to 301 in SEQ ID NO: 1 under stringent conditions, wherein the nucleotide corresponding to the nucleotide at position 290 is a cytosine and is labeled with a fluorescent dye. As referred to in this context, the hybridizing sequence hybridizes with the complementary strand of SEQ ID NO:1 or the recited portion of SEQ ID NO:1. Thus, said oligonucleotide has 12 to 54 nucleotides derived from said hybridizing sequence, including nucleotides corresponding to nucleotides 290 to 301 in SEQ ID NO:1. The 12 to 54 nucleotides are all contained, consecutively in SEQ ID NO:1 or the hybridizing sequence.
The (P2) and (P2') probes of the present invention may also be peptide nucleic acid (PNA) having amide-linked N-(2-aminoethyl)glycine as the backbone instead of the deoxyribose backbone of an oligonucleotide, or may be nucleic acid having two cyclic structures wherein the oxygen atom at the 2'-position and the carbon atom at the 4'-position in the sugar of nucleic acid are cross-linked (LNA; Locked Nucleic Acid).
For example, in the (P2) and (P2') probes of the present invention, the nucleotide at position 301 in SEQ ID NO:1 is A, which is the wild-type nucleotide.
The (P2) and (P2') probes of the present invention can be prepared in the same manner as the quenching probe described in JP 2002-119291 A except that the (P2) and (P2') probes of the present invention have the above-described specified sequence in the nucleotide sequence shown in SEQ ID NO:1. As shown in Table 3, the length of the probe of the present invention is, for example, 12 to 54 consecutive nucleotides, 12 to 30 consecutive nucleotides or 13 to 25 consecutive nucleotides.

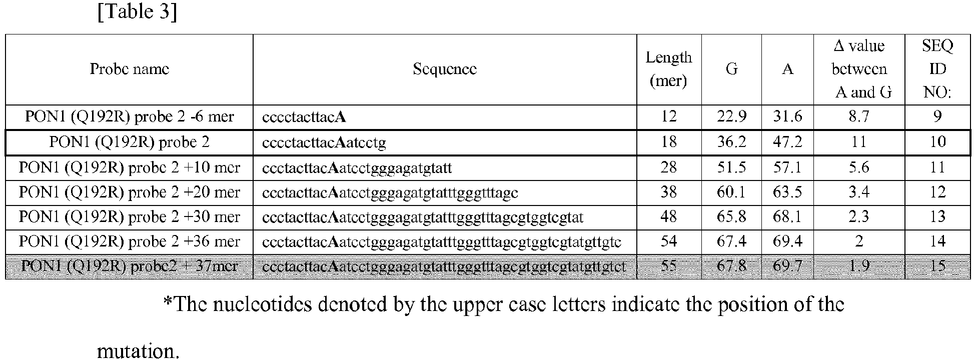

The Tm value was calculated with Meltcalc 99 free (http://www.meltcalc.com/) under the conditions of: Oligoconc [µM] 0.2, Na eq. [mM] 50.

In the present invention, for example, the mutation of the nucleotide at position 301 in SEQ ID NO:1 can be more sensitively detected in cases where the difference between the Tm value observed when the (P2) oligonucleotide is hybridized with DNA having the complementary nucleotide sequence and the Tm value observed when the (P2) oligonucleotide is hybridized with DNA having the complementary nucleotide sequence except that only one nucleotide at position 301 in SEQ ID NO:1 is not complementary is not less than 2.0°C.
Examples of the nucleotide sequence of the (P2) probe used in the present invention include 5'-cccctacttacAatcctg-3' (SEQ ID NO:10), in which the upper-case nucleotide A corresponds to the nucleotide at position 301.

The fluorescent dye is not restricted, and examples of the fluorescent dye include fluorescein, phosphor, rhodamine and polymethine dye derivatives. Examples of commercially available fluorescent dyes include BODIPY FL (trademark; manufactured by Molecular Probes), FluorePrime (trade name; manufactured by Amersham Pharmacia), Fluoredite (trade name; manufactured by Millipore), FAM (manufactured by ABI), Cy3 and Cy5 (manufactured by Amersham Pharmacia) and TAMRA (manufactured by Molecular Probes). The conditions for detection using the probe are not restricted, and may be appropriately determined depending on the fluorescent dye used. The detection may be carried out, for example, with a detection wavelength of 445 to 480 nm in the case of Pacific Blue, with a detection wavelength of 585 to 700 nm in the case of TAMRA, and with a detection wavelength of 520 to 555 nm in the case of BODIPY FL. By using such a probe, hybridization and dissociation can be easily confirmed based on fluctuation of the signal. The fluorescent dye can be bound to the oligonucleotide by a conventional method such as the method described in JP 2002-119291 A.

The "homologous sequence" in the present specification means a nucleotide sequence having homology (or identity) of, for example, not less than 80%, 81%, 82%, 83%, 84%, 85%, 86%, 87%, 88%, 89%, 90%, 91%, 92%, 93%, 94%, 95%, 96%, 97%, 98% or 99% to a particular nucleotide sequence. The identity may also be 100% in the present application.
The hybridization described herein can be carried out according to a known method or a method corresponding thereto, such as the method described in Molecular Cloning 3rd Edition (J. Sambrook et al., Cold Spring Harbor Lab. Press, 2001). Said hybridization is under stringent conditions, for example.
The term "stringent conditions" means conditions under which a specific hybrid is formed while non-specific hybrids are not formed. Typical examples of stringent conditions include conditions under which hybridization is performed with a potassium concentration of about 25 mM to about 50 mM and a magnesium concentration of about 1.0 mM to about 5.0 mM. Examples of the conditions in the present invention include conditions under which hybridization is performed in Tris-HCl (pH 8.6), 25 mM KCl and 1.5 mM MgCl₂, but the conditions are not limited thereto. Other examples of stringent conditions include those described in Molecular Cloning 3rd Edition (J. Sambrook et al., Cold Spring Harbor Lab. Press, 2001), and are hereby incorporated in the present specification by reference. Those skilled in the art can easily select such conditions by, for example, controlling the hybridization reaction and/or changing salt concentrations of the hybridization reaction solution.
Examples of the oligonucleotide of the above fluorescently labeled oligonucleotide include oligonucleotides as well as modified oligonucleotides.
Examples of the constituent units of the above oligonucleotide include ribonucleotides, deoxyribonucleotides and artificial nucleic acids. Examples of the artificial nucleic acids include DNA; RNA; LNA (Locked Nucleic Acid), which is an RNA analogue; PNA (Peptide Nucleic Acid), which is a peptide nucleic acid; and BNA (Bridged Nucleic Acid), which is a bridged nucleic acid.
The oligonucleotide may be constituted by either a single type of constituent unit or a plurality of types of constituent units among the above constituent units.

The (P1) and (P1') probes of the present invention have a nucleotide sequence of 6 to 53 consecutive nucleotide containing nucleotides 299 to 304 in SEQ ID NO:1, and have homology to SEQ ID NO:1 except that the nucleotide at position 304 is a cytosine. That is, although the (P1) and (P1') probes of the present invention are homologous to the nucleotide sequence of 6 to 53 consecutive nucleotides containing nucleotides 299 to 304, the probe does not need to be completely identical thereto.
The (P2) and (P2') probes of the present invention have a nucleotide sequence of 12 to 54 consecutive nucleotides containing nucleotides 290 to 301 in SEQ ID NO: 1, and have homology to SEQ ID NO:1 except that the nucleotide at position 290 is a cytosine. That is, although the (P2) and (P2') probe of the present invention are homologous to the nucleotide sequence of 12 to 54 consecutive nucleotides containing nucleotides 290 to 301 in SEQ ID NO:1, the probe does not need to be completely identical thereto.

For example, the fluorescently labeled oligonucleotide is a fluorescently labeled oligonucleotide whose fluorescence intensity decreases (or the fluorescence is quenched) or increases when the oligonucleotide is hybridized with a complementary sequence, relative to the fluorescence intensity observed when the oligonucleotide is not hybridized with the complementary sequence. For example, the fluorescence intensity of the fluorescently labeled oligonucleotide decreases when the oligonucleotide is hybridized with a complementary sequence, relative to the fluorescence intensity observed when the oligonucleotide is not hybridized with the complementary sequence. A probe that utilizes such a fluorescence quenching phenomenon is generally called a guanine quenching probe, and is known as a QProbe (registered trademark). For example, the fluorescently labeled oligonucleotide is an oligonucleotide which is designed such that the 3'-end or 5'-end of the oligonucleotide is C, which terminal C is labeled with a fluorescent dye such that the emission decreases when the terminal C comes into proximity with G.
Further, the probe of the present invention is, for example, labeled with a fluorescent dye at its 5'- or 3'-end.
In the present specification, when the term "first, second or third position counted from the 5'-end" is mentioned, the 5'-end is regarded as the first position. Further, when the term "first, second or third position counted from the 3'-end" is mentioned, the 3'-end is regarded as the first position.

In the detection method of the present invention, nucleic acid comprising the PON1 gene polymorphism (Q192R), and/or the CYP2C19*2 and/or CYP2C19*3 polymorphism(s) is analyzed using a nucleic acid probe labeled with a fluorescent dye(s) by measuring fluorescence from the fluorescent dye(s), to perform melting curve analysis, followed by detecting the polymorphism(s) based on the result of the melting curve analysis, which nucleic acid probe is the probe of the present invention.

The detection method of the present invention uses the probe of the present invention and comprises the following steps:
(I) adding the probe of the present invention to a sample containing DNA and allowing the probe to hybridize with the DNA;
(II) changing the temperature to dissociate the hybridization complex between the DNA and the probe and measuring the fluctuation of the signal due to dissociation of the hybridization complex;
(III) analyzing the fluctuation of the signal to determine the Tm value; and
(IV) determining based on the Tm value the presence or absence of the polymorphism of interest.

The evaluation of the Tm value in (III) includes not only evaluation of the dissociation temperature of the hybridization complex but also evaluation of the differential value of the fluorescence signal which fluctuates upon melting of the hybridization complex depending on the temperature.

Examples of the method of nucleic acid amplification include methods using a polymerase, such as PCR, ICAN and LAMP. When the amplification is carried out by a method using a polymerase, the amplification may be carried out in the presence of the probe of the present invention. Those skilled in the art can easily control the reaction conditions and the like of the amplification depending on the probe used. Accordingly, the detection can be carried out just by analyzing the Tm value of the probe after the amplification of the nucleic acid, so that the amplification product does not need to be handled after the reaction. Therefore, there is no risk of contamination of the amplification product. Further, since the detection can be carried out with the same apparatus as the one necessary for the amplification, it is not necessary to transfer the container. Therefore, automation can also be easily done.

As the DNA polymerase to be used for the PCR method, a conventional DNA polymerase may be used without any limitation. Examples of the DNA polymerase include GeneTaq (manufactured by Nippon Gene Co., Ltd.), PrimeSTAR Max DNA Polymerase (manufactured by Takara Bio Inc.) and Taq Polymerase.
The amount of the polymerase to be used is not restricted as long as the polymerase is used at a concentration which is normally employed. For example, in cases where Taq polymerase is used, the concentration of the polymerase may be 0.01 U to 100 U per 50 µl of the reaction solution. This has an advantage, for example, in that a sufficient amount of amplified product can be obtained.

In the present invention, the DNA in the sample may be either single-stranded DNA or double-stranded DNA. In cases where the DNA is double-stranded DNA, for example, the step of dissociating the double-stranded DNA in the sample by heating may be included before the hybridization step. By dissociating the double-stranded DNA into single-stranded DNA, hybridization with the fluorescently labeled oligonucleotide is possible.

In the present invention, the ratio (molar ratio) of the probe of the present invention to be added with respect to the DNA in the sample is not restricted, and the ratio is, for example, not more than 1 or not more than 0.3 with respect to the DNA in the sample to ensure a sufficient detection signal. In this case, for example, the DNA in the sample may be either: the sum of the target DNA wherein the polymorphism to be detected is present and non-target DNA wherein the polymorphism to be detected is absent; or the sum of the amplification product containing the target sequence wherein the polymorphism to be detected is present and the amplification product containing the non-target sequence wherein the polymorphism is absent. Although the ratio of the DNA to be detected relative to the DNA in the sample is usually not known, the ratio (molar ratio) of the probe to be added with respect to the DNA to be detected (the amplification product containing the sequence to be detected) is, for example, not more than 100, not more than 50, or not more than 30, as a result. The lower limit of the ratio is not restricted, and the ratio is, for example, not less than 0.001, not less than 0.01, or not less than 0.2. The ratio of the probe of the present invention to be added with respect to the DNA may be, for example, either the molar ratio with respect to the double-stranded DNA or the molar ratio with respect to the single-stranded DNA.

The Tm value will now be described. Heating a solution containing double-stranded DNA causes an increase in absorbance at 260 nm. This is caused because the hydrogen bonds between the strands of the double-stranded DNA are broken by the heat and the double-stranded DNA is dissociated into single-stranded DNA (melting of DNA). When the double-stranded DNA is completely dissociated into single-stranded DNA, the absorbance is about 1.5-fold higher than the absorbance at the beginning of heating (absorbance for only the double-stranded DNA), and completion of the melting can be judged accordingly. Based on this phenomenon, the melting temperature Tm is generally defined as the temperature at which the increase in absorbance reached 50% of the total increase in absorbance.

In the present invention, measurement of the signal fluctuation due to the temperature change for determination of the Tm value can also be carried out by measuring absorbance at 260 nm based on the above-mentioned principle, but the measurement may be carried out based on a signal from a label added to the probe of the present invention, which signal fluctuates depending on the state of hybridization between the DNA and the probe. Therefore, as the probe of the present invention, the above-mentioned labeled probe may be used. Examples of the labeled probe include a fluorescently labeled oligonucleotide probe which emits fluorescence when it is not hybridized with the target sequence, whose fluorescence intensity decreases (or the fluorescence is quenched) when the probe is hybridized with the target sequence, and a fluorescently labeled oligonucleotide probe which emits fluorescence when it is not hybridized with the target sequence, whose fluorescence intensity increases when the probe is hybridized with the target sequence. In the case of the former probe, the probe shows no signal or shows a weak signal when it forms a hybrid (double-stranded DNA) with the sequence to be detected, while the probe shows a signal or the signal increases when the probe is released by heating. In the case of the latter probe, the probe shows a signal when forming a hybrid (double-stranded DNA) with the sequence to be detected, while the signal decreases (disappears) when the probe is released by heating. Therefore, by detecting the change in the signal from the label under conditions specific to the signal (by investigating absorbance or the like), the progress of melting and the Tm value can be determined in a similar manner to the measurement of absorbance at 260 nm. For example, the labeling substance in the labeled probe is as mentioned above, and the probe may be labeled with a fluorescent dye.

The method of the present invention for detecting a polymorphism will now be described by way of specific examples of the method by detection of changes in the signal from a fluorescent dye. It should be noted that the method of the present invention for detecting a polymorphism is characterized by the use *per se* of the probe for detection of a polymorphism, and other steps and conditions are not restricted.

The temperature range at which fluctuation of the fluorescence intensity is measured is not restricted, and the starting temperature may be, for example, room temperature to 85°C, or 25°C to 70°C, and the end temperature may be, for example, 40°C to 105°C. The heating rate is not restricted, and may be, for example, 0.1°C/second to 20°C/second, or 0.3°C/second to 5°C/second.

The fluctuation of the signal is then analyzed to determine the Tm value. More particularly, the differential value at each temperature (-d the fluorescence intensity/dt) is calculated based on the obtained/measured fluorescence intensity, and the temperature at which the value is lowest may be determined as the Tm value. Further, the point at which the amount of increase in fluorescence intensity per unit time (the amount of increase in fluorescence intensity/t) is largest may also be determined as the Tm value. In cases where, as the labeled probe, a probe with which the signal intensity increases upon hybridization is used instead of a quenching probe, the amount of decrease in fluorescence intensity may be measured instead.

In the present invention, the hybridization complex is heated and fluorescence signal fluctuation (for example, an increase in fluorescence intensity) due to the increase in the temperature is measured as described above, but, instead of employing this method, signal fluctuation upon hybridization may be measured, for example. That is, fluorescence signal fluctuation caused by formation of the hybridization complex due to the decrease in the temperature of the sample to which the probe was added may be measured.

For example, in cases where a fluorescently labeled oligonucleotide probe whose fluorescence intensity is lower (or whose fluorescence is quenched) when the probe is hybridized with the complementary sequence than when the probe is not hybridized with the complementary sequence (e.g., QProbe) is used, the fluorescence intensity is high upon addition of the probe to the sample since the probe is in the dissociated state, while the fluorescence decreases (or is quenched) upon formation of a hybridization complex due to the decrease in the temperature. Therefore, for example, the temperature of the heated sample may be gradually decreased, to measure the decrease in fluorescence intensity due to the temperature decrease.
On the other hand, in cases where a labeled probe whose signal increases upon formation of the hybrid (e.g. TaqMan probe manufactured by Roche Molecular Systems, Inc.) is used, the fluorescence intensity is low (or the fluorescence is quenched) upon addition of the probe to the sample since the probe is in the dissociated state, while the fluorescence intensity increases upon formation of a hybridization complex due to the decrease in the temperature. Therefore, for example, the temperature of the sample may be gradually decreased, to measure the increase in fluorescence intensity due to the temperature decrease.

The nucleic acid to be used as a template for carrying out the nucleic acid amplification is not restricted as long as it contains nucleic acid, and examples of the nucleic acid include those derived from, or those which may be derived from, arbitrary biological origins such as blood; oral mucosal suspensions; somatic cells of nails, hair and the like; germ cells; milk; ascitic fluid; paraffin-embedded tissues; gastric juices; fluids obtained by gastric lavage; peritoneal fluid; amniotic fluid; and cell cultures. The nucleic acid as a template may be used as it is directly after being obtained from any of the above-mentioned origins, or may be pretreated to modify properties of the sample before being used. For example, in cases where whole blood is used as a sample, isolation of genomic DNA from the whole blood may be carried out by a known method. For example, a commercially available genomic DNA isolation kit (trade name, GFX Genomic Blood DNA Purification kit; manufactured by GE Healthcare Bio-Sciences KK) or the like may be employed.

The primer pair used in the PCR is not restricted as long as a region with which the probe of the present invention can be hybridized is amplified. Those skilled in the art can easily design such a primer pair based on the nucleotide sequence of SEQ ID NO:1. The length and the Tm value of each primer are usually 12-mer to 40-mer and 40 to 70°C, or 16-mer to 30-mer and 55 to 60°C, respectively.
The primers in the primer pair do not need to have the same length, but the Tm values of these primers are almost the same (or the difference is not more than 5°C), for example.

The Tm analysis can be carried out in the same manner as in the conventional method except that fluorescence from the fluorescent dye of the probe of the present invention is measured. The fluorescence can be measured using an excitation light having a wavelength dependent on the fluorescent dye while measuring light having an emission wavelength dependent on the fluorescent dye. The heating rate in the Tm analysis is usually 0.3 to 5°C/second. The composition of the reaction solution used for carrying out the Tm analysis is not restricted as long as the probe can hybridize with nucleic acid having the complementary sequence of the nucleotide sequence of the probe, and usually, the concentration of monovalent cations is 1.5 to 5 mM, and the pH is 7 to 9. Since the reaction solution in an amplification method using a DNA polymerase, such as PCR, usually satisfies these conditions, the reaction solution after the amplification can be used as it is for the Tm analysis.

Detection of the PON1 gene polymorphism (Q192R) and the CYP2C19*2 and CYP2C19*3 gene polymorphisms based on the result of the Tm analysis can be carried out according to a conventional method. The detection in the present invention includes detection of the presence/absence of a polymorphism (s).
Since detection of the presence/absence of a polymorphism (s) is possible with the probe and by the method of the present invention, judgment (or prediction) of the pharmacological effect of Clopidogrel and resistance to Clopidogrel based on the presence/absence of a polymorphism (s) is also included in the present invention. More specifically, in cases where the nucleotide at the PON1 gene polymorphism rs662 is the wild-type A (A/A), it is suggested that Clopidogrel is less likely to be effective and the risk of stent thrombosis is high, while in cases where the mutant type G (G/G) is contained, the risk is suggested to be low. Further, in cases where the CYP2C19*2(G681A) or CYP2C19*3(G636A) mutation is homozygous (that is, CYP2C19*2 is A/A or CYP2C19*3 is A/A), it is suggested that drugs such as Clopidogrel show decreased metabolic efficiencies and the risk of side effects is high.
Thus, by the present invention, all of the PON1 gene polymorphism (Q192R) and the CYP2C19*2 and CYP2C19*3 gene polymorphisms can be detected at once (i.e. can be detected simultaneously).

The polymorphism probes for the CYP2C9*2 and CYP2C9*3 mutation sites which can be used in simultaneous detection (that is, detection in the same system) of the PON1 gene polymorphism are described in, for example, JP 2005-58114 A, WO2008/066162 and JP 2005-110502 A. More specifically, each of the probes is a nucleic acid probe labeled with a fluorescent dye at the 5'-end or 3'-end, and fluorescence from the fluorescent dye decreases upon occurrence of hybridization. The nucleic acid probes have the nucleotide sequences shown in SEQ ID NO:64 and SEQ ID NO:76 in WO2008/066162. The nucleotide sequences shown in SEQ ID NO:64 and SEQ ID NO:76 in WO2008/066162 are the same as the nucleotide sequences shown in SEQ ID NO:24 and SEQ ID NO:28 in the present invention.

### <2> Kit of the Present Invention

The kit of the present invention is a kit to be used for the detection method of the present invention. This kit comprises a nucleic acid probe labeled with a fluorescent dye whose fluorescence changes upon hybridization (e.g., quenching probe), which probe is composed of the above-described oligonucleotide(s). The kit of the present invention may also be used for judging (or predicting) the pharmacological effect of Clopidogrel and resistance to Clopidogrel.

The detection kit (or reagent kit) of the present invention may further comprise, in addition to the probe, reagents required for nucleic acid amplification in the detection method of the present invention, especially a primer(s) for amplification using a DNA polymerase.
More specifically, the primers of the present invention are primers for amplifying a region in the nucleotide sequence shown in SEQ ID NO:1 in the PON1 gene, which region comprises a sequence with which the P1, P1', P2 or P2' oligonucleotide hybridizes (e.g., SEQ ID NOs:16 and 17). Primers for amplifying a region in the nucleotide sequence shown in SEQ ID NO:21 in the CYP2C19*2 gene, which region comprises a sequence with which the oligonucleotide shown in SEQ ID NO:24 hybridizes (e.g., SEQ ID NOs:22 and 23); and/or primers for amplifying a region in the nucleotide sequence shown in SEQ ID NO:25 in the CYP2C19*3 gene, which region comprises a sequence with which the oligonucleotide shown in SEQ ID NO:28 hybridizes (e.g., SEQ ID NOs:26 and 27); are also included in the present invention and the primers disclosed herein form a further aspect of the invention.

In the detection kit of the present invention, the probe, primer and other reagents may be stored separately, or a part of these components may be stored as a mixture(s).
The present invention will now be described concretely by way of Examples. However, these Examples are merely examples and the present invention is not restricted thereto.
In the present invention, in terms of the individual sequences of the sample nucleic acid in the sample to be subjected to the detection, and the probes and primers for detecting polymorphisms, matters described herein based on the complementary relationship between these sequences are applied to the respective sequences and also to the sequences complementary thereto unless otherwise specified. When the matters of the present invention are applied to the sequence complementary to each sequence, the sequence recognized by the complementary sequence is read as the sequence complementary to the corresponding sequence described in the present specification throughout the specification according to the common technical knowledge.

### EXAMPLES

### Example 1 (Use of a Single Probe for Detection of Template Oligonucleotide for PON1)

Based on the nucleotide sequence comprising the site of the PON1 gene polymorphism (Q192R) rs662 (SEQ ID NO:1), the probe shown in Table 4 having C at its 3'-end was designed. In Table 4, the position of the probe is represented by nucleotide positions in the nucleotide sequence shown in SEQ ID NO:1. Labeling with Pacific Blue was carried out according to a conventional method.

The sequences of the template oligonucleotides (wild-type (SEQ ID NO:18) and mutant type (SEQ ID NO:19)) used as the subject sequences to be detected are shown in Table 4. In Table 4, the positions of the oligonucleotides are represented by nucleotide positions in the nucleotide sequence shown in SEQ ID NO:1. In the case of the heterozygous type, the template oligonucleotides of SEQ ID NOs:18 and 19 were mixed at a ratio of 1:1 to provide the sample.

**[Table 4]**

| Name | Sequence (5'→3') | Position | SEQ ID NO: | mer | GC content (%) | Tm (mt) | Tm (WT) | ΔTm |
|---|---|---|---|---|---|---|---|---|
| PON1 (Q192R) probe | tgacccctacttac**G**atc-(Pacific Blue) | 287-304 | 2 | 18 | 50 | 48.6 | 41 | 7.6 |

| | | | | | | | | |
|---|---|---|---|---|---|---|---|---|
| *The nucleotide denoted by the upper case letter indicates the position of the mutation. | | | | | | | | |

| Template Oligonucleotide | Sequence (5'→3') | Position | SEQ ID NO: | mer |
|---|---|---|---|---|
| Wild-type | cccaggat**T**gtaagtaggggtcaag | 309-285 | 18 | 25 |
| Mutant-type | cccaggat**C**gtaagtaggggtcaag | 309-285 | 19 | 25 |

| | | | | |
|---|---|---|---|---|
| *The nucleotides denoted by the upper case letters indicate the position of the mutation. | | | | |

Using the reagents described below and a fully automatic SNPs testing device (trade name: i-densy (registered trademark), manufactured by ARKRAY, Inc.), Tm analysis was carried out to evaluate the performance of the fluorescently labeled oligonucleotide (SEQ ID NO:2).
In the Tm analysis, treatment was carried out at 95°C for 1 second and then at 40°C for 60 seconds, followed by increasing the temperature from 40°C to 75°C at a heating rate of 1°C/3 seconds, while measuring changes in fluorescence intensity with time. The excitation wavelength and detection wavelength in the Tm analysis were 365 to 415 nm and 445 to 480 nm, respectively (Pacific Blue).

**[Table 5]**

| (Reaction solution volume: 50 µl) | |
|---|---|
| 1 × Gene Taq Universal buffer (manufactured by Nippon Gene Co., Ltd.) | |
| PON1 (Q192R) probe | 0.2 µM |
| Template oligonucleotide | 0.2 µM |

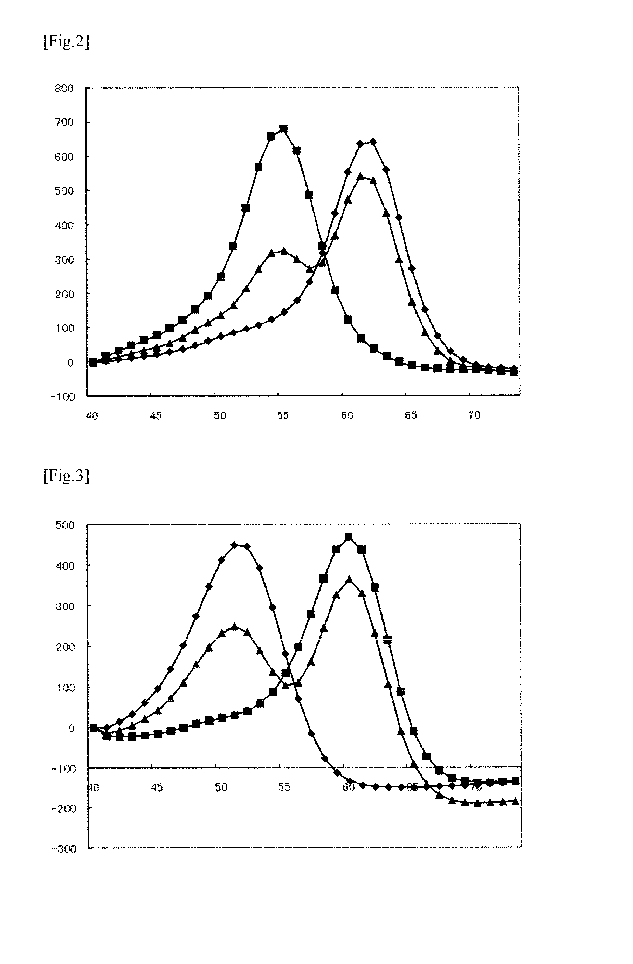

As a result of the Tm analysis using the probe shown in Table 4, the wild-type oligonucleotide (squares) showed a peak of Pacific Blue at about 55°C, and the mutant-type oligonucleotide (diamonds) showed a peak of Pacific Blue at about 62°C (Fig. 2). The heterozygous oligonucleotide (triangles) wherein the wild-type and the mutant type are mixed showed peaks of Pacific Blue at about 55°C and about 61 °C (Fig. 2).
As described above, it was revealed that the wild-type (A/A), heterozygous type (A/G) and mutant type (G/G) can be distinguished from one another since these clearly showed unique patterns of peaks.

### Example 2 (Use of a Single Probe for Detection of Template Oligonucleotide for PON1)

Based on the nucleotide sequence comprising the site of the PON1 gene polymorphism (Q192R) rs662 (SEQ ID NO:1), the probe shown in Table 6 having C at its 5'-end was designed. In Table 6, the position of the probe is represented by nucleotide positions in the nucleotide sequence shown in SEQ ID NO:1. Labeling with TAMRA was carried out according to a conventional method.

The sequences of the template oligonucleotides (wild-type (SEQ ID NO:18) and mutant type (SEQ ID NO:19)) in Table 4 were used as the subject sequences to be detected. In the case of the heterozygous type, the template oligonucleotides of SEQ ID NOs:18 and 19 were mixed at a ratio of 1:1 to provide the sample.

**[Table 6]**

| Name | Sequence (5'→3') | Position | SEQ ID NO: | mer | GC content (%) | Tm (mt) | Tm (WT) | ΔTm |
|---|---|---|---|---|---|---|---|---|
| PON (QI92R) probe2 | (TAMRA)-cccctacttacAatcctg-P | 290-307 | 10 | 18 | 50 | 36.2 | 47.2 | 11 |

| | | | | | | | | |
|---|---|---|---|---|---|---|---|---|
| *The nucleotide denoted by the upper case letter indicates the position of the mutation. P at the 3'-end represents phosphorylation. | | | | | | | | |

Using the reagents described below and a fully automatic SNPs testing device (trade name: i-densy (registered trademark), manufactured by ARKRAY, Inc.), Tm analysis was carried out to evaluate the performance of the fluorescently labeled oligonucleotide (SEQ ID NO:10).
In the Tm analysis, treatment was carried out at 95°C for 1 second and then at 40°C for 60 seconds, followed by increasing the temperature from 40°C to 75°C at a heating rate of 1°C/3 seconds, while measuring changes in fluorescence intensity with time. The excitation wavelength and detection wavelength in the Tm analysis were 520 to 555 nm and 585 to 700 nm, respectively (TAMRA).

**[Table 7]**

| (Reaction solution volume: 50 µl) | |
|---|---|
| 1 × Gene Taq Universal buffer (manufactured by Nippon Gene Co., Ltd.) | |
| PON1 (Q192R) probe 2 | 0.2 µM |
| Template oligonucleotide | 0.2 µM |

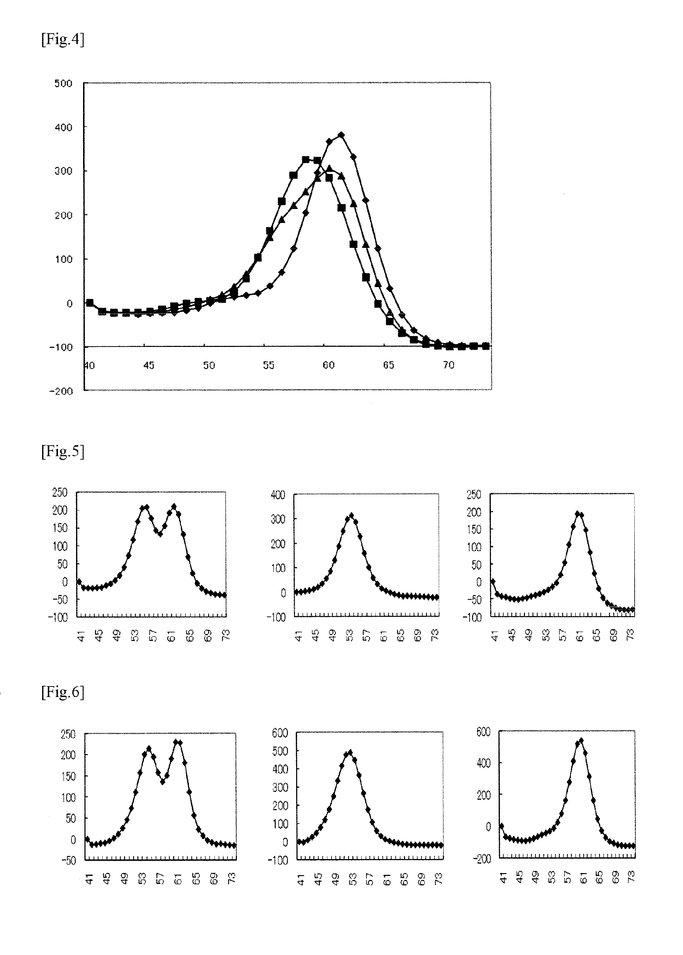

As a result of the Tm analysis using the probe shown in Table 6, the wild-type oligonucleotide (squares) showed a peak of TAMRA at about 60°C, and the mutant-type oligonucleotide (diamonds) showed a peak of TAMRA at about 52°C (Fig. 4). Therefore, it was revealed that the wild-type and the mutant type can be distinguished from each other.
The heterozygous oligonucleotide (triangles) wherein the wild-type and the mutant type are mixed showed peaks of TAMRA at about 51°C and about 60°C (Fig. 4). Therefore, it was revealed that the heterozygous type can be distinguished from the wild-type and the mutant type.

### Comparative Example 1 (Use of a Single Probe for Detection of Template Oligonucleotide for PON1)

Based on the nucleotide sequence comprising the site of the PON1 gene polymorphism (Q192R) rs662 (SEQ ID NO:1), the probe shown in Table 8 having C at its 5'-end was designed. In Table 8, the position of the probe is represented by nucleotide positions in the nucleotide sequence shown in SEQ ID NO:1. Labeling with TAMRA was carried out according to a conventional method.
The sequences of the template oligonucleotides in Table 4 (wild-type (SEQ ID NO:18) and mutant type (SEQ ID NO:19)) were used as the subject sequences to be detected. In the case of the heterozygous type, the template oligonucleotides of SEQ ID NOs:18 and 19 were mixed at a ratio of 1:1 to provide the sample.

**[Table 8]**

| Name | Sequence (5'→3') | Position | SEQ ID NO: | mer | GC content (%) | Tm (mt) | Tm (WT ) | ΔTm |
|---|---|---|---|---|---|---|---|---|
| PON1 (Q192R) probe 3 | (TAMRA)-cttgacccctacttacGa | 285-302 | 20 | 18 | 50 | 48.9 | 41.2 | 7.6 |

| | | | | | | | | |
|---|---|---|---|---|---|---|---|---|
| *The nucleotide denoted by the upper case letter indicates the position of the mutation. | | | | | | | | |

Using the reagents described below and a fully automatic SNPs testing device (trade name: i-densy (registered trademark), manufactured by ARKRAY, Inc.), Tm analysis was carried out to evaluate the performance of the fluorescently labeled oligonucleotide (SEQ ID NO:20).
In the Tm analysis, treatment was carried out at 95°C for 1 second and then at 40°C for 60 seconds, followed by increasing the temperature from 40°C to 75°C at a heating rate of 1°C/3 seconds, while measuring changes in fluorescence intensity with time. The excitation wavelength and detection wavelength in the Tm analysis were 520 to 555 nm and 585 to 700 nm, respectively (TAMRA).

**[Table 9]**

| (Reaction solution volume: 50 µl) | |
|---|---|
| 1 × Gene Taq Universal buffer (manufactured by Nippon Gene Co., Ltd.) | |
| PON1 (Q192R) probe 3 | 0.2 µM |
| Template oligonucleotide | 0.2 µM |

As a result of the Tm analysis using the probe shown in Table 8, the wild-type oligonucleotide (squares) showed a peak of TAMRA at about 60°C, and the mutant-type oligonucleotide (diamonds) showed a peak of TAMRA at about 61°C (Fig. 4). Therefore, it was revealed that these cannot be distinguished from each other because of the small Δ (difference) of the Tm value.
Further, the heterozygous oligonucleotide (triangles) wherein the wild-type and the mutant type are mixed showed a peak of TAMRA only at about 60°C (Fig. 4). Therefore, it was revealed that the heterozygous type shows only a single detection peak and cannot be distinguished from the wild-type and the mutant type.
Accordingly, taking the above results into account together with the results in Examples 1 and 2, it can be understood that the sequence of the probe cannot be arbitrary even in cases where C at the 5'-end or 3'-end of the probe is fluorescently labeled, and that fluorescent labeling of C at position 290 or 304 in the nucleotide sequence shown in SEQ ID NO:1, as is the cases of the probes shown in SEQ ID NOs:2 and 10, is important.

### Example 3 (Use of a Plurality of Probes for Detection in Purified Human Genomic DNA or Whole Blood)

Polymorphic regions were amplified as described below from purified human genomic DNA or whole blood by PCR using the primers described below, and Tm analysis was carried out using the probes shown in SEQ ID NOs:2, 24 and 28.
First, based on the nucleotide sequence comprising the site of the PON1 gene polymorphism (Q192R) rs662 (SEQ ID NO:1), the primers shown in Table 10 were designed such that the polymorphic site can be amplified. In Table 10, the positions of the primers are represented by nucleotide positions in the nucleotide sequence shown in SEQ ID NO:1.
Subsequently, based on the nucleotide sequence comprising the site of the CYP2C19*2 gene polymorphism (G681A) (SEQ ID NO:21), primers shown in Table 11 were designed such that the polymorphic site can be amplified. In Table 11, the positions of the primers are represented by nucleotide positions in the nucleotide sequence shown in SEQ ID NO:21. Position 200 in SEQ ID NO:21 is represented by r, which means that the wild-type nucleotide is G and the mutant-type nucleotide is A.
Further, based on the nucleotide sequence comprising the site of the CYP2C19*3 gene polymorphism (G636A) (SEQ ID NO:25), primers shown in Table 11 were designed such that the polymorphic site can be amplified. In Table 11, the positions of the primers are represented by nucleotide positions in the nucleotide sequence shown in SEQ ID NO:25. Position 302 in SEQ ID NO:25 is represented by r, which means that the wild-type nucleotide is G and the mutant-type nucleotide is A.
Thereafter, PCR and Tm analysis were carried out using a fully automatic SNPs testing device (trade name: i-densy IS-5310, manufactured by ARKRAY, Inc.). The composition of the PCR reaction solution was as described below. As a sample, whole blood or purified human genomic DNA as described below was used. The conditions for PCR and Tm analysis were: 95°C for 60 seconds→(95°C for 1 second →66°C for 30 seconds) X 50 cycles→95°C for 1 second→40°C for 60 seconds→ (40°C→75°C, 1°C/3 seconds).
The excitation wavelength and detection wavelength in the Tm analysis were 365 to 415 nm and 445 to 480 nm, respectively (Pacific Blue); 420 to 485 nm and 520 to 555 nm, respectively (BODIPY FL); or 520 to 555 nm and 585 to 700 nm, respectively (TAMRA).

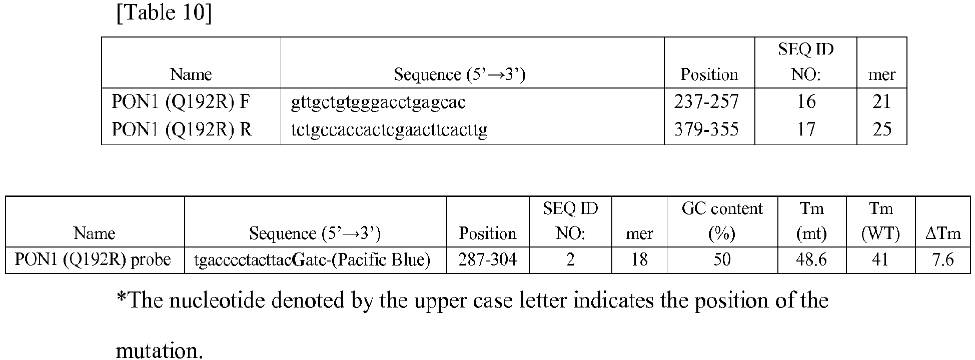

**[Table 11]**

| Name | Sequence (5'→3') | Position | SEQ ID NO: | mer |
|---|---|---|---|---|
| CYP2C19(*2) F | taaattattgttttctcttagatatgcaataattttccca | 141-180 | 22 | 40 |
| CYP2C19(*2) R | cccgagggttgttgatgtccatc | 302-280 | 23 | 23 |
| CYP2C19(*2) probe | tccc**A**ggaacccataac-(BODIPY FL) | 196-212 | 24 | 17 |

| | | | | |
|---|---|---|---|---|
| *The nucleotide denoted by the upper case letter indicates the position of the mutation. | | | | |

| Name | Sequence (5'→3') | Position | SEQ ID NO: | mer |
|---|---|---|---|---|
| CYP2C19(*3) F | tgatggaaaaattgaatgaaaacatcaggattgta | 256-290 | 26 | 35 |
| CYP2C19(*3) R | tcaaaaatgtacttcagggcttggtcaata | 407-378 | 27 | 30 |
| CYP2C19(*3) probe | (TAMRA)-ccctg**A**atccaggtaag-**P** | 297-313 | 28 | 17 |

| | | | | |
|---|---|---|---|---|
| *The nucleotide denoted by the upper case letter indicates the position of the mutation. P at the 3'-end represents phosphorylation. | | | | |

**[Table 12]**

| <<In cases where purified human genomic DNA is used as the template>> | |
|---|---|
| (Reaction solution volume: 50 µl) | |
| 1 × PCR buffer | |
| dNTP | 0.2 mM |
| MgCl₂ | 4.5 mM |
| Taq polymerase (manufactured by ARKRAY, Inc.) | 1.88 U/test |
| PON1 (Q192R) F | 0.3 µM |
| PON1 (Q192R) R | 1.5 µM |
| CYP2C19(*2) F | 0.3 µM |
| CYP2C19(*2) R | 1.5 µM |
| CYP2C19(*3) F | 0.5 µM |
| CYP2C19(*3) R | 2.5 µM |
| PON1 (Q192R) probe | 0.2 µM |
| CYP2C19(*2) probe | 0.2 µM |
| CYP2C19(*3) probe | 0.2 µM |
| Purified human genomic DNA* | 100 copies |

| | |
|---|---|
| *Human genomic DNA purified from whole blood is used. | |

**[Table 13]**

| <<In cases where whole blood is used as the template>> | |
|---|---|
| (Reaction solution volume: 50 µl) | |
| 1 × PCR buffer | |
| dNTP | 0.2 mM |
| MgCl₂ | 4.5 mM |
| Taq polymerase (manufactured by ARKRAY, Inc.) | 1.88 U/test |
| PON1 (Q192R) F | 0.3 µM |
| PON1 (Q192R) R | 1.5 µM |
| CYP2C19(*2) F | 0.3 µM |
| CYP2C19(*2) R | 1.5 µM |
| CYP2C19(*3) F | 0.5 µM |
| CYP2C19(*3) R | 2.5 µM |
| PON1 (Q192R) probe | 0.2 µM |
| CYP2C19(*2) probe | 0.2 µM |
| CYP2C19(*3) probe | 0.2 µM |
| Pretreated whole blood | (4 µl*) |

| | |
|---|---|
| *Pretreated whole blood is used. | |

### <Purified DNA>

Purified DNA was added to the PCR reaction solution such that 100 copies/test of the DNA was contained therein, and used as a template.

### <Preparation of Whole Blood>

To 70 µl of dilution buffer (1), 10 µl of whole blood was added, and the resulting mixture was mixed well, followed by adding 10 µl of the resulting mixture to 70 µl of dilution buffer (2). By heating 17 µl of this mixture at 95°C for 10 minutes, 4 µl of pretreated whole blood was obtained. The pretreated whole blood was added to the PCR reaction solution, to use DNA originating from the pretreated whole blood as a template.

**[Table 14]**

| Dilution buffer (1) | |
|---|---|
| Tris-HCl (pH 8.0) | 10 mM |
| EDTA (pH 8.0) | 0.1 mM |
| SDS | 0.30% |
| | |

| Dilution buffer (2) | |
|---|---|
| Tris-HCl (pH 8.0) | 10 mM |
| 500 mM EDTA (pH 8.0) | 0.1 mM |

As a result of the evaluation of the PON1 gene with the fluorescence of Pacific Blue, the peaks for both the wild-type and the mutant type were found for the purified human genomic DNA (Fig. 5, left panel) and the blood (Fig. 6, left panel). Thus, the samples were revealed to have the heterozygous genotype (A/G).
As a result of the evaluation of the CYP2C19*2 gene with the fluorescence of BODIPY FL, only the peak for the wild-type was found for the purified human genomic DNA (Fig. 5, middle panel) and the blood (Fig. 6, middle panel). Thus, the samples were revealed to have the wild-type genotype (G/G).
As a result of evaluation of the CYP2C19*3 gene with fluorescence of TAMRA, only the peak for the mutant type was found for the purified human genomic DNA (Fig. 5, right panel) and the blood (Fig. 6, right panel). Thus, the samples were revealed to have the mutant-type genotype (A/A).

As can be seen from the results shown in Figs. 5 and 6, when the probes shown in SEQ ID NOs:2, 24 and 28 were used, changes in fluorescence intensity that can be analyzed by Tm analysis were observed for the PON1 gene polymorphism and the CYP2C19*2 and CYP2C19*3 gene polymorphisms.
That is, in terms of the PON1 gene polymorphism, the purified human genomic DNA and the whole blood having the heterozygous genotype A/G showed two peaks (at about 56°C and about 62°C) and hence exhibited a unique pattern in respect of the amount of change in fluorescence intensity. Further, in terms of the CYP2C19*2 gene polymorphism, the purified human genomic DNA and the whole blood showed a single peak (about 54°C, wild-type G/G) and hence exhibited a unique pattern in respect of the amount of change in fluorescence intensity. Further, also in terms of the CYP2C19*3 gene polymorphism, the purified human genomic DNA and the whole blood showed a single peak (about 62°C, mutant type A/A) and hence exhibited a unique pattern of the amount of change in fluorescence intensity.
Thus, using the probes of SEQ ID NOs:2, 24 and 28 simultaneously, the PON1 gene polymorphism and the CYP2C19*2 and CYP2C19*3 gene polymorphisms can be detected at once (i.e. simultaneously).
Further, since the probe of SEQ ID NO:10 is labeled at C at its end as in the case of the probe of SEQ ID NO:2 and its effect has been demonstrated in Example 2, it is also possible to detect the PON1 gene polymorphism and the CYP2C19*2 and CYP2C19*3 gene polymorphisms at once by simultaneous use of the probe of SEQ ID NO:10 and the probes of SEQ ID NOs:24 and 28.

### INDUSTRIAL APPLICABILITY

The present invention can be suitably used in fields such as healthcare, diagnosis and research.

## Claims

1. A probe for detecting a polymorphism in the PON1 gene, comprising at least one fluorescently labeled oligonucleotide selected from P1, P1', P2 and P2' below:
(P1) an oligonucleotide comprising a nucleotide sequence of 6 to 53 consecutive nucleotides containing nucleotides 299 to 304 in SEQ ID NO: 1 or a homologous sequence thereof, wherein the nucleotide corresponding to the nucleotide at position 304 is a cytosine and is labeled with a fluorescent dye;
(P1') an oligonucleotide comprising a nucleotide sequence of 6 to 53 consecutive nucleotides containing nucleotides 299 to 304 in SEQ ID NO: 1 or a nucleotide sequence which hybridizes with the complementary strand of the nucleotides 299 to 304 in SEQ ID NO: 1 under stringent conditions, wherein the nucleotide corresponding to the nucleotide at position 304 is a cytosine and is labeled with a fluorescent dye;
(P2) an oligonucleotide comprising a nucleotide sequence of 12 to 54 consecutive nucleotides containing nucleotides 290 to 301 in SEQ ID NO: 1 or a homologous sequence thereof, wherein the nucleotide corresponding to the nucleotide at position 290 is a cytosine and is labeled with a fluorescent dye; and
(P2') an oligonucleotide comprising a nucleotide sequence of 12 to 54 consecutive nucleotides containing nucleotides 290 to 301 in SEQ ID NO:1 or a nucleotide sequence which hybridizes with the complementary strand of the nucleotides 290 to 301 in SEQ ID NO: 1 under stringent conditions, wherein the nucleotide corresponding to the nucleotide at position 290 is a cytosine and is labeled with a fluorescent dye.

2. The probe according to claim 1, wherein
said oligonucleotides P1 and P1' have the nucleotide corresponding to the nucleotide at position 304 labeled with a fluorescent dye at the first, second or third position counted from the 3'-end; and
said oligonucleotides P2 and P2' have the nucleotide corresponding to the nucleotide at position 290 labeled with a fluorescent dye at the first, second or third position counted from the 5'-end.

3. The probe according to claim 1 or 2, wherein
said oligonucleotides P1 and P1' have the nucleotide corresponding to the nucleotide at position 304 labeled with a fluorescent dye at the 3'-end; and
said oligonucleotides P2 and P2' have the nucleotide corresponding to the nucleotide at position 290 labeled with a fluorescent dye at the 5'-end.

4. The probe according to any one of claims 1 to 3, wherein said fluorescently labeled oligonucleotide emits fluorescence when the fluorescently labeled oligonucleotide is not hybridized with a target sequence, and the fluorescence intensity decreases or increases when the fluorescently labeled oligonucleotide is hybridized with a target sequence.

5. The probe according to claim 4, wherein said fluorescently labeled oligonucleotide emits fluorescence when the fluorescently labeled oligonucleotide is not hybridized with a target sequence, and the fluorescence intensity decreases when the fluorescently labeled oligonucleotide is hybridized with a target sequence.

6. The probe according to any one of claims 1 to 5, wherein
said oligonucleotides P1 and P1' have 6 to 28 consecutive nucleotides, preferably 6 to 18 consecutive nucleotides; and
said oligonucleotides P2 and P2' have 12 to 28 consecutive nucleotides, preferably 12 to 18 consecutive nucleotides.

7. The probe according to any one of claims 1 to 6, wherein said probe is a probe for melting curve analysis.

8. A method for detecting a polymorphism in the PON1 gene in a sample containing nucleic acid which may contain said polymorphism comprising contacting the probe according to any one of claims 1 to 7 with said sample and detecting the presence or absence of said polymorphism.

9. The method according to claim 8, comprising Steps (I) to (IV) below:
(I) adding said probe according to any one of claims 1 to 7 to a sample containing DNA and allowing said probe to hybridize with said DNA;
(II) changing the temperature to dissociate the hybridization complex between said DNA and said probe and measuring the fluctuation of the signal due to said dissociation of said hybridization complex;
(III) analyzing said fluctuation of the signal to determine the Tm value; and
(IV) determining based on said Tm value the presence of the polymorphism of interest,
wherein preferably the nucleic acid is amplified before said Step (I) or at the same time as said Step (I).

10. The method according to claim 8 or 9, further comprising detecting in the same system at least one polymorphism selected from a polymorphism of the nucleotide corresponding to the nucleotide at position 200 in SEQ ID NO:21 and a polymorphism of the nucleotide corresponding to the nucleotide at position 302 in SEQ ID NO:25.

11. The method according to any one of claims 8 to 10, comprising detecting in the same system a polymorphism of the nucleotide corresponding to the nucleotide at position 301 in SEQ ID NO:1, a polymorphism of the nucleotide corresponding to the nucleotide at position 200 in SEQ ID NO:21 and a polymorphism of the nucleotide corresponding to the nucleotide at position 302 in SEQ ID NO:25.

12. A method for judging the pharmacological effect of a drug in a subject, comprising detecting a polymorphism in the PON1 gene by the method for detecting a polymorphism according to any one of claims 8 to 11 and judging resistance to said drug and/or the pharmacological effect of said drug based on the presence or absence of said polymorphism in a sample from said subject.

13. A reagent kit for detecting a polymorphism in the PON1 gene, comprising the probe according to any one of claims 1 to 7, and preferably further comprising a primer for amplifying a nucleotide sequence comprising the region with which said probe hybridizes.

14. The kit according to claim 13, further comprising:
a polymorphism detection probe for detecting a polymorphism of the nucleotide corresponding to the nucleotide at position 200 in SEQ ID NO:21; and
a polymorphism detection probe for detecting a polymorphism of the nucleotide corresponding to the nucleotide at position 302 in SEQ ID NO:25.
